# EUROPEAN PATENT APPLICATION

(11) **EP 4 749 668 A1**
(43) Date of publication of application: **27.05.2026**
(21) Application number: 25218426.2
(22) Date of filing: 25.11.2025
(51) Int. Cl.: H01F 27/40, H02H 5/04, H02H 6/00, H02H 7/04

(54) **A SYSTEM AND METHOD FOR MONITORING LOAD USAGE ON AN ELECTRICAL TRANSFORMER**

(30) Priority: 25.11.2024 NL 2039160
(71) Applicant: Eskom Holdings SOC Limited, 2000 Sunninghill, Sandton (ZA)
(72) Inventor: DE KLERK, Nicolaas, 2000 Sunninghill, Sandton (ZA)
(74) Representative: WSL Patentanwälte Partnerschaft mbB

(57) **Abstract**

The present invention relates to a system and method which is capable of monitoring temperature changes over time on a region of an electrical transformer to measure load usage and thereby prevent any damage thereto.

## Description

### FIELD OF APPLICATION OF THE INVENTION

This invention relates to a system and method for monitoring load usage on an electrical power transformer. More particularly, the inventor relates to a system and method for preventing damage to an electrical power transformer.

### BACKGROUND TO THE INVENTION

Transformers are crucial to the distribution and transmission of electrical energy, have evolved significantly since their inception in the late 19th century. Initially conceived as static devices for voltage transformation in alternating current (AC) systems, transformers have undergone substantial advancements to meet the growing demands of modern power networks. The evolution of transformers can be delineated into several key stages, each marked by innovations aimed at enhancing efficiency, reliability, and safety.

The inception of transformers can be traced back to the pioneering work of Michael Faraday and others in the early 19th century on electromagnetic induction. Faraday's discovery laid the foundation for the development of transformers by demonstrating the principle of inducing a voltage in a coil through a changing magnetic field. Building upon Faraday's findings, the first practical transformer was devised by William Stanley in 1886, enabling the transmission of alternating current over long distances by stepping up voltages for reduced losses. Throughout the early 20th century, transformer technology advanced steadily, driven by the burgeoning electricity industry. Innovations such as the introduction of core materials with higher magnetic permeability and improved insulation materials contributed to increased efficiency and reliability. The development of oil-immersed transformers in the 1920s further improved cooling and insulation, allowing for higher power ratings and longer service life. The mid-20th century witnessed significant strides in transformer design and manufacturing techniques. The introduction of controlled cooling methods, such as oil forced-air and oil forced-water cooling, enabled transformers to handle higher loads while maintaining optimal operating temperatures. Additionally, the advent of solid-state electronics in the latter half of the century facilitated the integration of monitoring and protection systems into transformer designs, enhancing operational safety and reliability.

In recent decades, the evolution of transformers has been shaped by advancements in materials science, computational modelling, and digital technology. The emergence of amorphous and nano-crystalline core materials has led to transformers with reduced losses and improved energy efficiency. Furthermore, the integration of sensors, communication protocols, and advanced analytics has enabled real-time monitoring and predictive maintenance, minimising downtime and optimizing asset performance. Despite these advancements, transformers still face certain inherent disadvantages, particularly concerning overloading and illegal connections. Overloading occurs when transformers are subjected to excessive electrical loads beyond their rated capacity, leading to overheating and potential failure. Illegal connections, such as unauthorized tapping or tampering with transformer wiring, pose safety hazards and can compromise the integrity of the electrical system. Many existing solutions for addressing overloading and illegal connections rely on reactive measures, such as overcurrent protection devices and periodic inspections. While these measures are effective to some extent, they lack dynamism and proactive intervention capabilities. External protection devices can easily be bypassed to prevent the illegal connections from tripping the overcurrent protection devices. Gradual overloading of the transformer will not necessarily blow a medium voltage fuse causing the transformer to fail due to overloading.

CN111584216B discloses a transformer temperature monitoring and early-warning system including multiple oil and infrared temperature sensors, a controller with storage/timing/calculation and open-circuit protection modules, and threshold-based alerting and power-off actions (e.g., alarms at elevated temperatures and power cutoff above 95°C), along with display/remote communication and black-box recording; however, CN111584216B does not disclose the features of: (A) executing a closed action to close the electrical circuit on the basis of a determination that calculated temperature data is below a lower predetermined threshold value; and (B) executing an open action to open the electrical circuit on the basis of calculated temperature data exceeding an upper predetermined threshold value together with an elapsed-time condition exceeding a predetermined time threshold value.

US2491338A discloses a protective device for electrical apparatus such as transformers employing a vacuum switch with thermally actuated mechanism (expansible bellows with heater) to interrupt fault currents and respond to long-time overloads, providing repeated automatic opening and reclosing with snap-action operation and thermal lag; however, US2491338A does not disclose the features of: (A) executing a closed action to close the electrical circuit on the basis of a determination that calculated temperature data is below a lower predetermined threshold value; and (B) executing an open action to open the electrical circuit on the basis of calculated temperature data exceeding an upper predetermined threshold value together with an elapsed-time condition exceeding a predetermined time threshold value.

Given the above, it is clear that there exists a present need for a solution that is a dynamic and proactive solution that mitigate risks and ensure the integrity of power networks by protecting an electrical transformer in real time that is at risk of being damaged.

### OBJECT OF THE INVENTION

Accordingly, it is an object of the present invention to provide a system and method which is capable of monitoring load usage on an electrical transformer and thereby prevent any damage thereto.

### SUMMARY OF THE INVENTION

According to a first aspect thereof, there is provided a system for monitoring temperature and thereby preventing damage to an electrical transformer comprising an electrical circuit, the system comprising:
- a control unit comprising a memory and a processor, wherein the memory is capable of storing predetermined threshold values and storing a set of instructions which are executable by the processor to allow for a plurality of calculations to be performed;
- a heat sensor communicatively coupled to the memory of the control unit, wherein the heat sensor is locatable on a region of the electrical transformer for deriving temperature signals measured over time when the temperature changes in the region of the transformer, and wherein the heat sensor is communicatively coupled to an electrical device for converting the temperature signals measured over time to temperature data that is stored on the memory; and
- execution means communicatively coupled to the memory and the processor of the control unit for executing a closed action when a calculation is performed that yields a result where the temperature data is below a lower predetermined threshold value, and executing an open action when a calculation is performed that yields a result where the temperature data is above an upper predetermined threshold value, wherein the execution means is capable of being electrically connected to the electrical circuit to close the electrical circuit when the closed action is executed and open the electrical circuit when the open action is executed.

The temperature data may be comprised of one or more temperature reading value and one or more time reading value. The time reading value may comprise a time-of-day value and a date value. The memory may be selected from the group consisting of a non-transitory storage medium, transitory storage medium, or combinations thereof.

The execution means may be capable of executing the open action when a first condition and a second condition is achieved, wherein the first condition is achieved when the processor performs a calculation and the result is that the temperature reading value exceeds the upper predetermined threshold value, and the second condition is achieved when the processor performs a calculation and the result is that the time reading value exceeds the predetermined time threshold value.

The time reading value may be an elapsed time value that is calculated by a countdown module stored on the memory. The countdown module may be configured to initiate and calculate the elapsed time value when temperature reading value exceeds the upper predetermined threshold value. The open action may be executed when the elapsed time value exceeds the predetermined time threshold value.

The upper predetermined threshold, lower predetermined threshold value, and predetermined time threshold value may be adjustable according to the configuration of each electrical transformer and the ambient conditions where it operates.

The upper predetermined threshold value may be between 80 and 100 degrees Celsius. The lower predetermined threshold value may be between 40 and 60 degrees Celsius. The predetermined time threshold value may be between 10 and 20 minutes.

The region may be an oil level of the electrical transformer. The system may further comprise an oil monitoring device which is capable of receiving a sample of the oil, a sample of gas generated by the oil, or a sample comprising the oil and the gas generated by the oil in combination, wherein the oil monitoring device is capable of analysing the sample to derive concentration values and ratio values of the one or more chemical compounds in the sample. The oil monitoring device may be a Dissolved Gas Analysis (DGA) device. The open action may be executed when the concentration values and ratio values exceed predetermined concentration threshold values and predetermined ratio threshold values stored on the memory.

The system may comprise transmission means for transmitting the temperature data and action data derived when one or more actions are executed. The transmission means may include, but is not limited to Bluetooth, radio waves, microwaves, fibre optical cables, local area network and/or wide area network.

The temperature data and the action data may be capable of being transmitted to an external device comprising a graphical user interface for visualising the data, input means for feeding instructions to the graphical user interface, and an analytics module stored on the external device comprising a plurality of analytics tools which allows for data analysis of the data. The data may be analysed to derive information about the status of the electrical transformer.

The information about the status of the electrical transformer may comprise information about whether the electrical transformer is energised, de-energised, or it requires maintenance. The information indicating that the electrical transformer requires maintenance may result or be caused by a condition selected from the group consisting of a faulty heat sensor, the execution means not executing a closed action, no data received from the oil monitoring device, or combinations thereof.

The set of instructions may be one or more algorithm. The algorithm may be selected from the group consisting of a machine learning algorithm, an artificial intelligence algorithm, a deep learning algorithm, a heuristic algorithm, or combinations thereof. The system may comprise a model that is capable of being trained by the one or more algorithm comparing data to benchmark data stored on the memory to derive an output data set having predictive information about when the electrical transformer will require maintenance.

The heat sensor may be selected from the group consisting of a resistance temperature detector (RTD), a temperature sensor, a thermocouple, or combinations thereof. The heat sensor may be a PT100 temperature sensor. The system may further comprise one or more additional heat sensors locatable on one or more regions of the electrical transformer for deriving temperature signals measured over time. A plurality of temperature signals generated from heat sensors located at different regions may fed to the electrical device, which is capable of performing a calculation to derive more accurate temperature data from the plurality of temperature signals.

The system may further comprise one or more photo sensors that are communicatively coupled to the a control unit, and wherein the photo sensor is locatable within the electrical transformer. The photo sensors may be capable of deriving photo data when light is detected in the electrical transformer. The execution means may execute the open action when the photo data is above a predetermined threshold. The photo sensor may be a light sensor or a photodetector selected from the group consisting of photodiodes, phototransistors, light-dependent resistors (LDRs), and combinations thereof.

The electrical device may be an analogue-to-digital converter (ADC) for converting the temperature signals into discrete temperature data, or an operational amplifier used with an analogue comparator for converting the temperature signals into discrete temperature data.

The execution means may be a contactor for making or breaking the electrical connection in the electrical circuit. The execution means may be selected from the group consisting of a solid state relay, a circuit breaker with a close coil, a circuit breaker with a trip coil, circuit breaker with an automatic spring rewind function, or combinations thereof.

The control unit may further comprise a graphical user interface for displaying the temperature data derived from temperature signals measured over time. The control unit may further comprise input means communicatively coupled to the graphical user interface which allows for a user to provide input instructions to access and/or modify the temperature data.

The control unit may further comprise input means communicatively coupled to the graphical user interface which allows for a user to provide input instructions.

The system may further comprise a tamper-proof enclosure for preventing unauthorised access to the system and its components.

According to a second aspect of the invention, there is provided a method of preventing damage to an electrical transformer, the method comprising the steps of:
(i) communicatively coupling a control unit comprising a processor and a memory to an electrical transformer, wherein the memory is capable of storing predetermined threshold values and a set of instructions which are executable by the processor to allow for a plurality of calculations to be performed;
(ii) providing a heat sensor that is communicatively coupled to the memory of the control unit, wherein the heat sensor is locatable in a region of the electrical transformer for deriving temperature signals measured over time when the temperature changes in the region of the transformer, and wherein the heat sensor is communicatively coupled to an electrical device for converting the temperature signals measured over time to temperature data that is stored on the memory, and wherein the temperature data comprises temperature measurement values and time measurement values;
(iii) communicatively coupling execution means to the memory and the processor of the control unit, wherein the execution means is capable of being electrically connected to the electrical circuit to close the electrical circuit when a closed action is executed and open the electrical circuit when an open action is executed;
(iv)executing a timer action optionally with a countdown module stored on the memory when the temperature data exceeds an upper predetermined threshold value, wherein the countdown module allows for an elapsed time value to be calculated;
(v) executing the closed action optionally when a calculation is performed by the processor that yields a result where the temperature data is below a lower predetermined threshold value; and
(vi)executing an open action optionally when a calculation is performed by the processor that yields a result where the temperature data is above an upper predetermined threshold value and the elapsed time value exceeds a predetermined time threshold value stored on the memory.

The method may comprise a further step of generating action data when one or more of the open action, closed action, or timer action are executed. The action data may comprise data selected from the group consisting of the type of action executed, the time of day when the action is executed, the date when the action is executed, or combinations thereof. The method may comprise a step of training a model by having one or more algorithms stored on the memory for comparing the action data to benchmark data stored on the memory to derive an output data set having predictive information about when the electrical transformer will require maintenance. The model may be stored on a storage medium, which allows for the model to be applied on one or more data sets that are communicatively coupled to the memory.

The term communicatively coupled shall refer to, but not be limited to, the exchange of information, commands, electrical signals, or data between devices or platforms.

The above and other characteristics, features and advantages of the present invention will become apparent from the following detailed description, taken in conjunction with the accompanying drawing which illustrate, by way of example, the principles of the invention. This description is given for the sake of example only, without limiting the scope of the invention. The reference figures quoted below refer to the attached drawings.

### BRIEF DESCRIPTION OF THE DRAWINGS

Preferred embodiments of the invention are described below with reference to the accompanying figures, wherein:
- **Figure 1**: is a photograph of the control unit utilized in the invention;
- **Figure 2a**: is a photograph of a portion of a 100 kVA transformer;
- **Figure 2b**: is a photograph of the 100 kVA transformer illustrating the outputs of the contactor;
- **Figure 2c**: is a photograph of the interior region of the LV Cubicle of the 100 kVA transformer, where the Cubicle is a LV kiosk forming part of the transformer tank design;
- **Figure 3**: is a photograph of a temperature measurement device used to measure the temperature of the oil level of the electrical transformer;
- **Figure 4**: is a series of graphical user interface displays showing the settings viewable on the control unit;
- **Figure 5**: is a photograph of an external testing device comprising a graphical user interface for displaying readings and setting of the control unit;
- **Figure 6**: is a temperature controller circuit diagram;
- **Figure 7**: is a temperature controller printed circuit board (PCB); and
- **Figure 8**: is an internal electrical schematic of a self-protected transformer.

The presently disclosed subject matter will now be described more fully hereinafter with reference to the accompanying Examples, in which representative embodiments are shown. The presently disclosed subject matter can, however, be embodied in different forms and should not be construed as limited to the embodiments set forth herein. Rather, these embodiments are provided so that this disclosure will be thorough and complete, and will fully convey the scope of the embodiments to those skilled in the art.

### DESCRIPTION OF PREFERRED EMBODIMENTS OF THE INVENTION

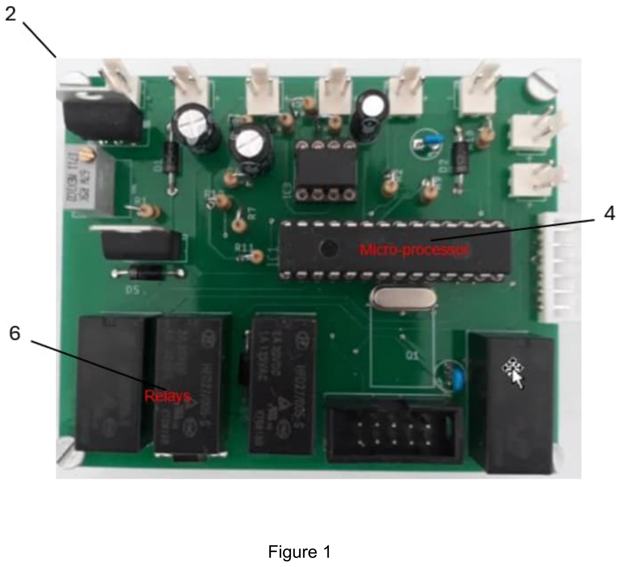

A non-limiting example of a preferred embodiment of the invention is described in more detail below, with reference to Figures 1 to 8.

With reference to Figure 1, there is provided a control unit 2 comprising a processor 4 and relays 6. The processor 4 is capable of performing a plurality of actions, which include an action of generating a signal to actuate a contactor (not shown) connected to the electrical circuit of an electrical transformer to either make or break the electrical circuit to control the flow of electrical power. The relays 6 are configured as electromechanical switching device that are used to control the flow of electrical power to various components in the control unit 2 and the electrical transformer.

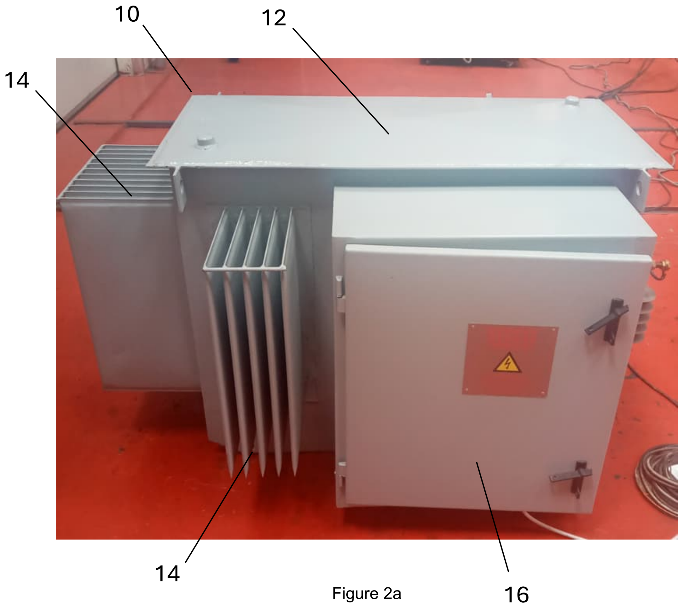

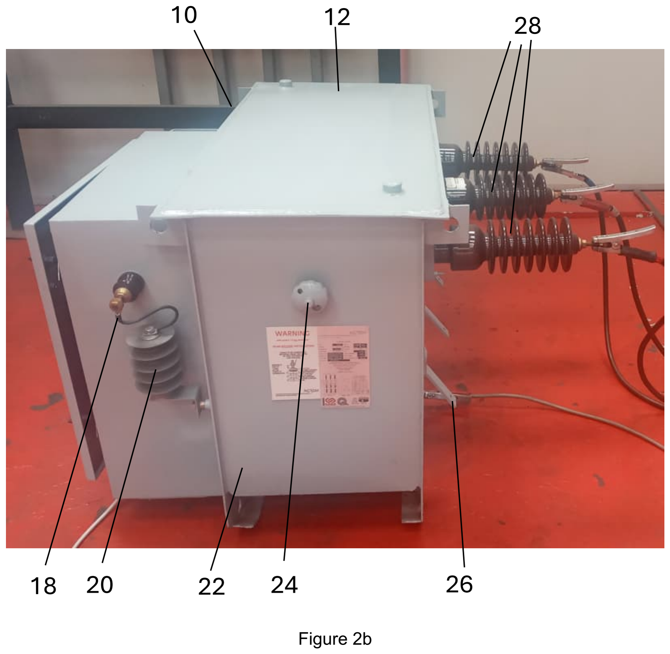

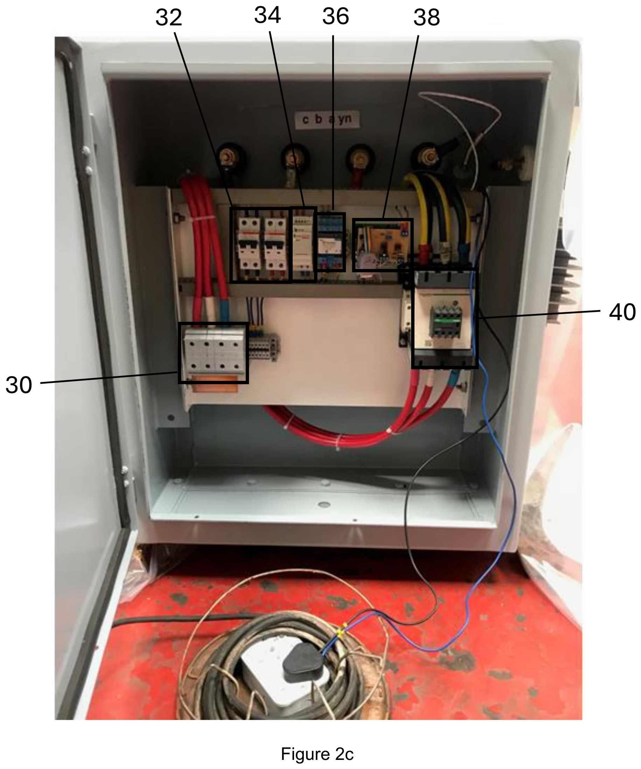

With reference to Figure 2a, there is provided an electrical transformer 10 (100kVA) having an enclosure 12 for enclosing a control unit, low voltage bushings, contactor and a sensor device operating as a thermocouple (not shown). The transformer 10 further includes corrugated radiators 14 and a LV Cubicle 16 containing LV bushings and control equipment (not shown). With reference to Figure 2b, there is provided the same electrical transformer 10 (100kVA) comprising an external neutral bushing 16, neutral surge arrestor 20, transformer tank 22, tap switch handle 24, MV surge arrestor support 26, and MV bushings 28. With reference to Figure 2c, the interior region of the LV Cubicle 16 is shown comprising elements such as the LV Cable Termination Block 30, LV Circuit breakers 32, 12V power supply unit 34, 220V Control Contactor 36, Electronic Control Unit (ECU) 38, and LV Power Contactor 40.

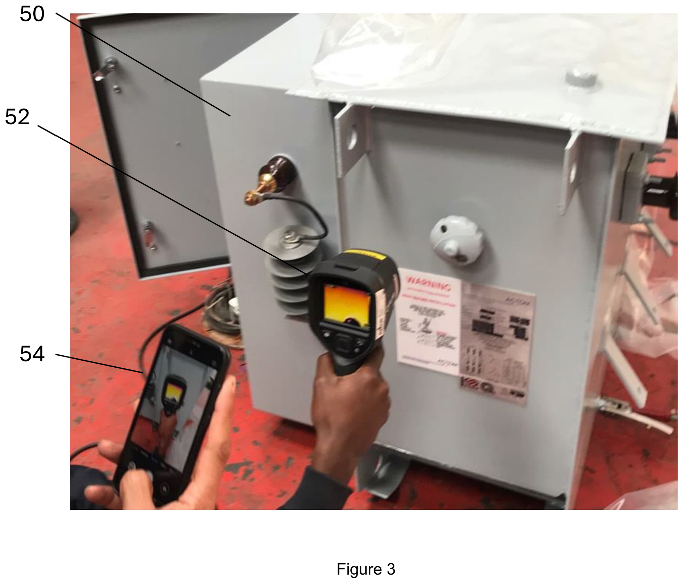

With reference to Figure 3, there is provided an electrical transformer 50, where a sensor device 52 is used to measure the temperature of the top level of the oil in the electrical transformer 50. The sensor device 52 may be a PT100 temperature sensor. The temperature reading is transmitted to a mobile device 54, which is capable of deriving temperature data to be transmitted to the control unit. Moreover, the mobile device 54 is capable of changing one or more predetermined settings to be compatible with a different environment or location. While not illustrated in this figure, the invention may include a photo sensor that is locatable in the electrical transformer, where the photo sensor is capable of detecting light or an arc, whereafter a data signal is immediately transmitted to the control unit to initiate a trip action.

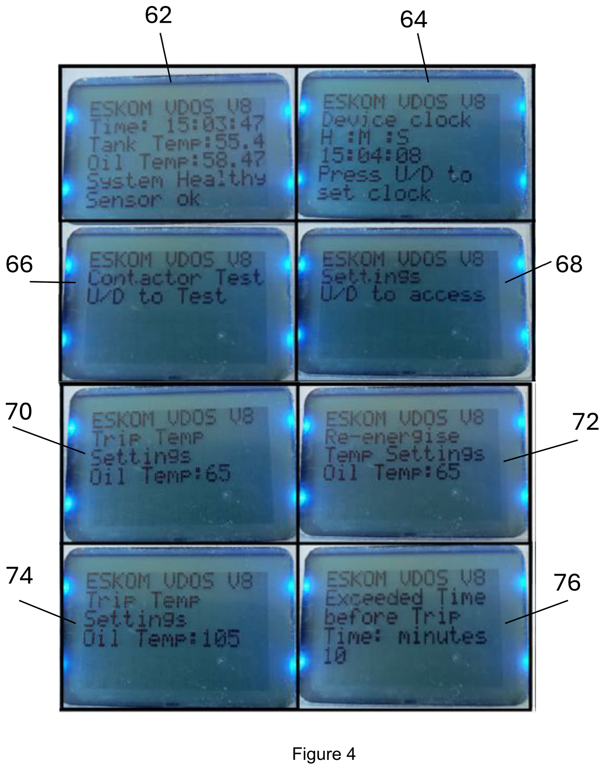

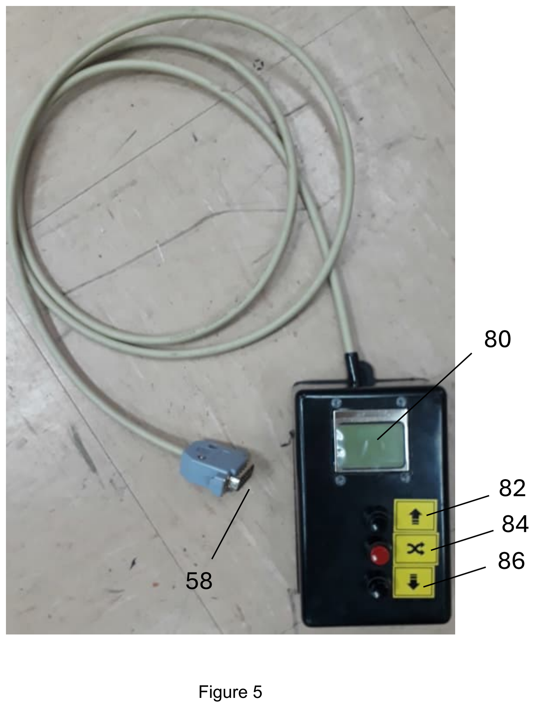

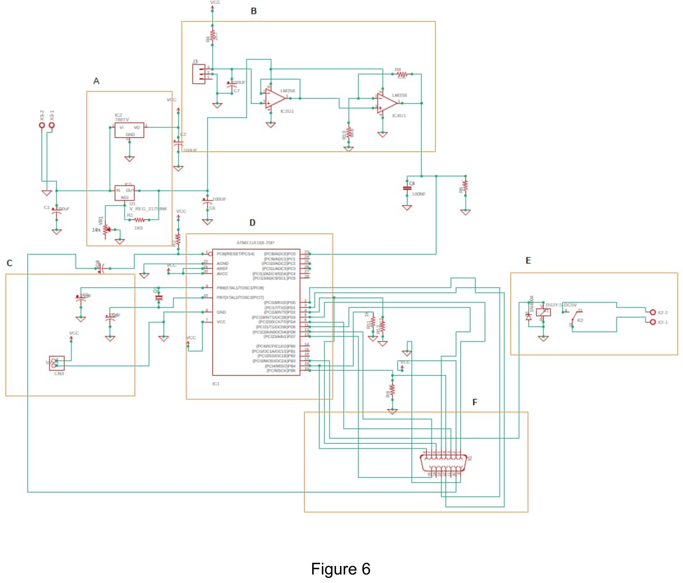

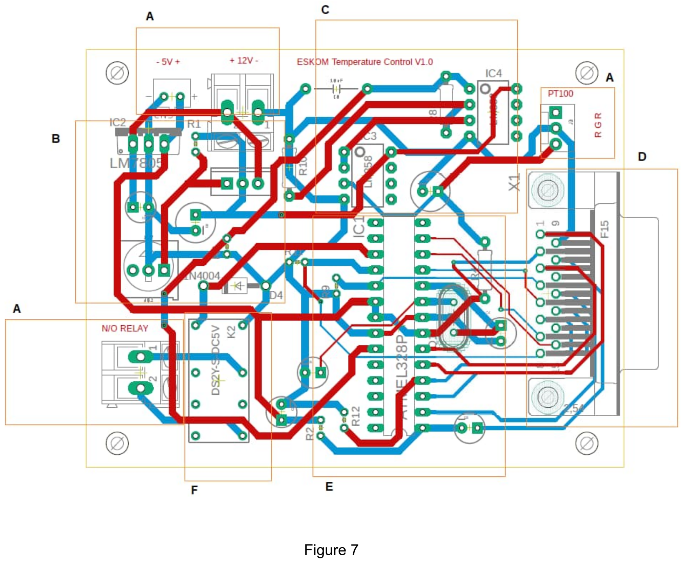

With reference to Figure 4, there is provided a plurality of displays of the user interface provided with the control unit. A first display 62 provides a breakdown of measured values, which importantly includes a current time measurement and temperature measurements of a tank (not shown) holding oil of the electrical transformer. A second display 64 provides a display where a user can adjust the time and clock. A third display 66 provides a display where a user can provide an instruction to execute a contactor test. A fourth display 68 provides a display where a user can modify the control unit settings. A fifth display 70 provides a display where a user can view the trip temperature associated with the oil temperature where the contactors are actuated to trip the electrical circuit and the electrical transformer to safeguard it from damage. A sixth display 72 provides a display where a user can view the re-energise temperature associated with the oil temperature where the contactors re-engage and complete the electrical circuit to allow for the flow of electrons. This temperature should be low enough where significant cooling has taken place to safeguard the electrical transformer from damage. A seventh display 74 provides a display where a user can view the trip temperature associated with the oil temperature where the contactors are actuated to trip the electrical circuit and the electrical transformer to safeguard it from damage. An eighth display 76 provides a display showing the time in minutes that needs to elapse before a trip action is executed. Figure 5 is a photo of the user interface 80 on a device connected to a control unit (not shown), wherein the user interface 80 is capable of displaying the various displays provided in Figure 4. The device comprises a plurality of buttons for adjusting the temperature of a transformer. These buttons include an increase temperature 82, stabilise temperature 84, and decrease temperature 86. A connector 58 is capable of connecting to a control unit provided on the transformer.
A. With reference to Figure 6, there is provided a temperature controller circuit diagram of the invention, which comprises 6 main modules as follows:A power supply regulated to fix voltage points using linear regulators (for example a fixed voltage regulator such as LM7805 and an adjustable voltage regulator such as LM317T).
B. Operational amplifier circuit converting the thermocouple change in resistance to a measurable linear voltage to be fed into the 5V Analog-to-Digital Converter ("ADC").
C. Auxiliary stable 5V output for transmission means, e.g. a GSM modem.
D. ATMEL Processor running the embedded firmware and controlling the Input/Output's, where the ATMEL processor may be replaced with any similar processor, i.e. AMD, Intel, and the like.
E. Output relay powering the contactor coil.
F. D15 port as a D-sub connector with the standard protocol for serial communication RS232 communication for firmware upgrade or handheld display/programming device.
A. With reference to Figure 7, there is provided a temperature controller printed circuit board (PCB) comprising the following components:2 or 3 pin connectors used for external connections, where any other 2 or 3 pin connectors can be used for this application rated according to the correct voltage specification.
B. Linear voltage regulators used for the software application for regulating temperature of the transformer. Any form of voltage regulation can be used including Pulse Width Modulation ("PWM") or any form of switch mode power supply to regulate the set voltages.
C. LM358 operational amplifiers used to convert the thermocouple resistance to a measurable voltage. Any other method of linear voltage conversion can be used or any other operational amplifier doing a similar function.
D. A D15 Input/Output connector, or any form of 15 pin connector can be used to perform a similar function.
E. An ATMEL 328P Processor used to process the information and control the required Input/Output's. Any other processor can be used with at least 1 ADC, serial communication and 8 digital Input/Output ports.
F. A 5 Volt relay with a contactor specification of 250V, 10A. Any other form of relay can be used correctly rated for the main contactor coil voltage and current.

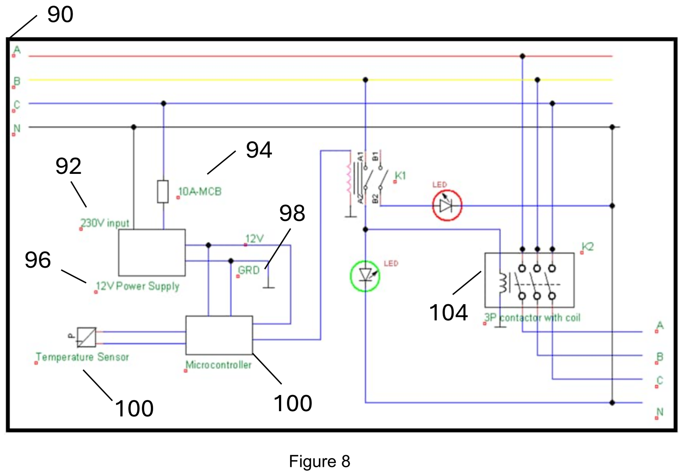

With reference to Figure 8, there is provided an internal electrical schematic of a self-protected transformer 90 comprising a 230V input 92, a 10 Ampere Miniature Circuit Breaker (10A-MCB) 94, a 12V power supply 96, a grounding 98, microcontroller 100, temperature sensor 102, and a 3-phase contactor with coil 104.

### Advantages:

The invention as described above is hereafter referred to as a self-protected transformer. Additional components are added to a typical electrical transformer to modify the same to yield the self-protected transformer. The approximate costs of these components are set out below for a 100kVA and 200 kVA transformer.

### Cost Analysis:

### 100kVA Transformer

| **Description** | **Unit** | **Qty** | **Item Cost** | **Total Cost** |
|---|---|---|---|---|
| Transformer 100kVA | Each | 1 | R 49 160 | R 49 160 |
| High risk enclosure | Each | 1 | R 10 100 | R 10 100 |
| Din rail | M | 0,5 | R 192 | R 96 |
| Contactor- 3P 150A 400V | Each | 1 | R 4 134 | R 4134 |
| 220V AC to 12V DC Din Rail Power Supply | Each | 1 | R 292 | R 292 |
| Electronic Control Unit | Each | 1 | R 650 | R 650 |
| Temperature Sensor | Each | 1 | R 470 | R 470 |
| Normally Opened: 12V coil 240V relay-Din rail | Each | 1 | R 257 | R 257 |
| Din rail Socket for 12V relay | Each | 1 | R 137 | R 137 |
| 10A LV breaker | Each | 1 | R 1 168 | R 1 168 |
| Din Rail PCB mount | Each | 1 | R 14 | R 14 |
| RED 220V Pilot light | Each | 1 | R 79 | R 79 |
| Green 220V Pilot light | Each | 1 | R 79 | R 79 |
| Contactor cable termination shroud | Each | 6 | R 102.50 | R 615 |
| CABLE .75kV 1C 70.0SQ CU FLEX D8026-(SAP-186800) | M | 3 | R 150 | R 450 |
| LUG, CRIMP CU 70.0SQxM12 F/H D3102 (SAP-0404422) | Each | 6 | R 9.67 | R 58 |
| **Total** | | | | **R 67 759** |
| **Additional Cost%** | | | | **38%** |

### 200kVA Transformer:

| **Description** | **Unit** | **Qty** | **Item Cost** | **Total Cost** |
|---|---|---|---|---|
| Transformer 200kVA | Each | 1 | R 69 006 | R 69 006 |
| High risk enclosure | Each | 1 | R 10 100 | R 10 100 |
| Din rail | M | 0,5 | R 192 | R 96 |
| Contactor- 3P 300A 400V | Each | 1 | R 7 224 | R 7 224 |
| 220V AC to 12V DC Din Rail Power Supply | Each | 1 | R 292 | R 292 |
| Electronic Control Unit | Each | 1 | R 650 | R 650 |
| Temperature Sensor | Each | 1 | R 470 | R 470 |
| Normally Opened: 12V coil 240V relay-Din rail | Each | 1 | R 257 | R 257 |
| Din rail Socket for 12V relay | Each | 1 | R 137 | R 137 |
| 10A LV breaker | Each | 1 | R 1 168 | R 1 168 |
| Din Rail PCB mount | Each | 1 | R 14 | R 14 |
| RED 220V Pilot light | Each | 1 | R 79 | R 79 |
| Green 220V Pilot light | Each | 1 | R 79 | R 79 |
| Contactor cable termination shroud | Each | 6 | R 102.50 | R 615 |
| CABLE .75kV 1C 70.0SQ CU FLEX D8026-(SAP-186800) | M | 3 | R 150 | R 450 |
| LUG, CRIMP CU 70.0SQxM12 F/H D3102 (SAP-0404422) | Each | 6 | R 9.67 | R 58 |
| **Total** | | | | **R 90 695** |
| **Additional Cost:** | | | | **31%** |

It is clear from the above tables that the self-protected transformer will be approximately 30 to 40 percent more expensive than the traditional transformer without any of the control technologies and tamper proof enclosure. It should be noted that the cost will decrease significantly with the increase on the transformer size as mainly the same equipment is used as set out in the tables above. Importantly, the additional cost quickly becomes insignificant in the long term when compared to the costs of non-technical losses, operational cost to manually reset circuit breakers due to overload, as well as replacement cost due to the damaged of equipment. It is therefore evident that the present invention provides an alternative solution whereby the longevity/lifetime of transformers are extended, while minimising the additional costs of having manual inspections and repairs done by having an automated solution that detects faults in real-time and executing necessary actions to allow the electrical transformer to normalise before resuming its operation.

## Claims

1. A system for monitoring temperature and thereby preventing damage to an electrical transformer (10) comprising an electrical circuit, the system comprising:
- a control unit (2) comprising a memory and a processor (4), wherein the memory is capable of storing predetermined threshold values and storing a set of instructions which are executable by the processor (4) to allow for a plurality of calculations to be performed;
- a heat sensor (52) communicatively coupled to the memory of the control unit (2), wherein the heat sensor (52) is locatable on a region of the electrical transformer (10) for deriving temperature signals measured over time when the temperature changes in the region of the transformer (10), and wherein the heat sensor (52) is communicatively coupled to an electrical device for converting the temperature signals measured over time to temperature data that is stored on the memory; and
- execution means (40) communicatively coupled to the memory and the processor (4) of the control unit (2) for executing a closed action when a calculation is performed that yields a result where the temperature data is below a lower predetermined threshold value, and executing an open action when a calculation is performed that yields a result where the temperature data is above an upper predetermined threshold value, wherein the execution means (40) is capable of being electrically connected to the electrical circuit to close the electrical circuit when the closed action is executed and open the electrical circuit when the open action is executed.

2. The system according to claim 1 wherein the temperature data comprises a temperature reading value and a time reading value, and wherein the execution means (40) is capable of executing the open action when a first condition and a second condition is achieved, wherein the first condition is achieved when the processor (4) performs a calculation and the result is that the temperature reading value exceeds the upper predetermined threshold value, and the second condition is achieved when the processor (4) performs a calculation and the result is that the time reading value exceeds the predetermined time threshold value.

3. The system according to claim 1, wherein the upper predetermined threshold, lower predetermined threshold value, and predetermined time threshold value are adjustable according to the configuration of each electrical transformer (10) and the ambient conditions where it operates.

4. The system according to claim 3 wherein the upper predetermined threshold value is between 80 and 100 degrees Celsius, the lower predetermined threshold value is between 40 and 60 degrees Celsius, and the predetermined time threshold value is between 10 and 20 minutes.

5. The system according to claim 1 wherein the region is where an oil level of the electrical transformer is located.

6. The system according to claim 1 further comprising an oil monitoring device which is capable of receiving a sample of the oil, a sample of gas generated by the oil, or a sample comprising the oil and the gas generated by the oil in combination, and wherein the oil monitoring device is capable of analysing the sample to derive concentration values and ratio values of the one or more chemical compounds in the sample, and wherein the execution means (40) executes an open action when the concentration values and ratio values exceed predetermined concentration threshold values and predetermined ratio threshold values stored on the memory.

7. The system according to claim 1 further comprises transmission means for transmitting the temperature data and action data derived when one or more closed action or one or more open action is executed.

8. The system according to claim 7 wherein the temperature data and the action data are capable of being transmitted to an external device (80) comprising a graphical user interface (80) for visualising the data, input means for feeding instructions to the graphical user interface (80), and an analytics module stored on the external device (80) comprising a plurality of analytics tools which allows for data analysis of the data.

9. The system according to claim 8 wherein data is analysed to derive information about the status of the electrical transformer (10), wherein the information comprises whether the electrical transformer (10) is energised, de-energised, or requires maintenance, and wherein the information indicating that the electrical transformer (10) requires maintenance results from a condition selected from the group consisting of a faulty heat sensor (52), the execution means (40) not executing a closed action, no data received from the oil monitoring device, or combinations thereof

10. The system according to claim 1 wherein the heat sensor (52) is selected from the group consisting of a resistance temperature detector (RTD), a temperature sensor, a thermocouple, or combinations thereof.

11. The system according to claim 1 further comprising one or more additional heat sensors locatable on one or more regions of the electrical transformer (10) for deriving a plurality of temperature signals measured over time, wherein the plurality of temperature signals generated are fed to the electrical device, which is capable of performing a calculation to derive more accurate temperature data from the plurality of temperature signals.

12. The system according to claim 1 further comprising one or more photo sensors that are communicatively coupled to the control unit (2), and wherein the photo sensor is locatable within the electrical transformer (10), wherein the one or more photo sensors are capable of deriving photo data when light is detected in a region of the electrical transformer (10), and wherein the execution means (40) executes an open action when the photo data is above a predetermined threshold.

13. The system according to claim 1 wherein the electrical device is an analogue-to-digital converter (ADC) or an operational amplifier.

14. The system according to claim 1 wherein the execution means (40) is a contactor for making or breaking the electrical connection in the electrical circuit, wherein the contactor is selected from the group consisting of a solid state relay, a circuit breaker with a close coil, a circuit breaker with a trip coil, a circuit breaker with an automatic spring rewind function, or combinations thereof.

15. The system according to claim 1 wherein the control unit (2) further comprises a graphical user interface (80) for displaying the temperature data derived from temperature signals measured over time, wherein the control unit (2) further comprises input means communicatively coupled to the graphical user interface (80) which allows for a user to provide input instructions.

16. The system according to claim 1 further comprising a tamper-proof enclosure (12) for preventing unauthorised access thereto.

17. The system according to claim 1, wherein the memory is selected from the group consisting of a non-transitory storage medium, transitory storage medium, or combinations thereof.

18. A method of preventing damage to an electrical transformer (10) comprising an electrical circuit, the method comprising the steps of:
(i) communicatively coupling a control unit (2) comprising a processor (4) and a memory to an electrical transformer (10), wherein the memory is capable of storing predetermined threshold values and a set of instructions which are executable by the processor (4) to allow for a plurality of calculations to be performed;
(ii) providing a heat sensor (52) that is communicatively coupled to the memory of the control unit (2), wherein the heat sensor (52) is locatable in a region of the electrical transformer (10) for deriving temperature signals measured over time when the temperature changes in the region of the transformer (10), and wherein the heat sensor (52) is communicatively coupled to an electrical device for converting the temperature signals measured over time to temperature data that is stored on the memory, and wherein the temperature data comprises temperature measurement values and time measurement values;
(iii) communicatively coupling execution means (40) to the memory and the processor (4) of the control unit (2), wherein the execution means (40) is capable of being electrically connected to the electrical circuit to close the electrical circuit when a closed action is executed and open the electrical circuit when an open action is executed;
(iv)executing a timer action with a countdown module stored on the memory when the temperature data exceeds an upper predetermined threshold value, wherein the countdown module allows for an elapsed time value to be calculated;
(v) executing the closed action when a calculation is performed by the processor (4) that yields a result where the temperature data is below a lower predetermined threshold value; and
(vi)optionally executing an open action when a calculation is performed by the processor (4) that yields a result where the temperature data is above an upper predetermined threshold value and the elapsed time value exceeds a predetermined time threshold value stored on the memory.

19. The method according to claim 18 comprising a further step of generating action data when one or more open action, closed action, or timer action is executed, wherein the action data comprises information selected from the group consisting of the type of action executed, the time of day when the action is executed, the date when the action is executed, or combinations thereof.

20. The method according to claim 19 further comprising a step of training a model by having one or more algorithms stored on the memory for comparing the action data to benchmark data stored on the memory to derive an output data set having predictive information about when the electrical transformer (10) will require maintenance, and storing the model on a storage medium, which allows for the model to be applied on one or more data sets that are communicatively coupled to the memory.
